# EUROPÄISCHE PATENTANMELDUNG

(11) **EP 4 574 137 A1**
(43) Veröffentlichungstag der Anmeldung: **25.06.2025**
(21) Anmeldenummer: 23218109.9
(22) Anmeldetag: 19.12.2023
(51) Int. Cl.: A61K 9/20, A61K 38/44

(54) **HERSTELLUNGSVERFAHREN FÜR EIN STABILISIERTES DIAMINOXIDASE-LYOPHILISAT UND PHARMAZEUTISCHE DARREICHUNGSFORM**

(71) Anmelder: STADA ARZNEIMITTEL AG, 61118 Bad Vilbel (DE)
(72) Erfinder: FORSTER, Florian, 3424 Zeiselmauer (AT); GIRSTMAIR, Sandra, 3430 Tulln (AT); LINDER, Angela, 3434 Unteroberndorf (AT)
(74) Vertreter: SONN Patentanwälte GmbH & Co KG

(57) **Zusammenfassung**

Offenbart wird ein Verfahren zur Herstellung eines stabilisierten Lyophilisats mit pflanzlicher Diaminooxidase (DAO), umfassend die folgenden Schritte: Bereitstellen von DAO-haltigen Pflanzen oder DAO-haltigen Teilen davon; Homogenisieren der DAO-haltigen Pflanzen oder DAO-haltigen Teilen davon in wässriger Lösung, um ein wässriges Homogenisat zu erhalten; Hinzufügen von (a) einem Zucker oder Zuckeralkohol und (b) einer Aminosäure als Stabilisator; und Lyophilisieren des wässrigen Homogenisats oder einer DAO-haltigen Fraktion davon in Anwesenheit des Stabilisators. Ebenfalls offenbart wird ein nach diesem Verfahren erhältliches Lyophilisat, sowie eine feste pharmazeutischen Darreichungsform zur oralen Verabreichung, die dieses Lyophilisat enthält. Diese Darreichungsform ist zur therapeutischen Verwendung, vorzugsweise in der Behandlung von Histamin-induzierten Störungen, vorgesehen bzw. kann sie als Nahrungsergänzungsmittel oder diätetisches Lebensmittel verwendet werden.

## Beschreibung

Die vorliegende Erfindung betrifft ein Verfahren zur Herstellung eines stabilisierten Lyophilisats mit pflanzlicher Diaminooxidase (DAO), sowie ein stabilisiertes Lyophilisat mit pflanzlicher DAO.

Histamin (2-(4-Imidazolyl)ethylamin) ist ein biogenes Amin, das im Organismus bei vielen physiologischen Prozessen beteiligt ist, aber auch in einigen Lebensmitteln vorkommt. Histamin kann bei Personen, die an einer Histamin-Unverträglichkeit leiden, verschiedene, oft unspezifische Symptome, die häufig Ähnlichkeit mit allergischen Reaktionen haben, auslösen.

Hohe Konzentrationen von freiem zirkulierendem Histamin können unter anderem Symptome wie Kopfschmerzen, verlegte bzw. rinnende Nase, Atemwegsobstruktionen, Tachykardie sowie Extrasystolen, weiters Magen- und Darmbeschwerden, die zu weichem Stuhl bis Durchfällen führen können, und Hypotonie, auslösen. Oft werden auch Schwellungen der Augenlider, gelegentlich auch urticarielle Exantheme beschrieben. Des Weiteren können Hautrötungen, Blutdruckabfall und Bronchospasmen auftreten.

DAO (EC 1.4.3.22) ist ein Enzym, das in verschiedenen Pflanzen, Tieren und Mikroorganismen vorkommt und für den Abbau von biogenen Aminen, insbesondere Histamin, verantwortlich ist. DAO wird auch als Histaminase bezeichnet.

Im Stand der Technik sind verschiedene DAO-haltige Präparate bekannt, die von an Histamin-Unverträglichkeiten leidenden Personen zu den Mahlzeiten eingenommen werden können, um den Abbau von Histamin aus der Nahrung im Darm zu unterstützen und dadurch die unerwünschten Symptome, die durch das über die Nahrung aufgenommene Histamin verursacht werden, zu verringern oder ganz zu verhindern.

So offenbart die WO 2006/003213 A1 pharmazeutische Zusammensetzungen, Nahrungsergänzungsmittel und kosmetische Zusammensetzungen umfassend DAO und deren Verwendung. Insbesondere wird die DAO magensaftresistent formuliert.

Tierische Quellen für die DAO sind bevorzugt, insbesondere DAO aus Schweinenieren.

Allerdings haben in den letzten Jahren auch DAO-Präparate auf pflanzlicher Basis an Bedeutung gewonnen. Unter anderem ist die allgemeine Akzeptanz von pflanzlichen Arzneimitteln und Nahrungsergänzungsmitteln höher, insbesondere unter Personen mit ethischen oder religiösen Bedenken bezüglich der Verwendung von tierischen Produkten. Außerdem kann die Gewinnung von DAO aus nachhaltig angebauten Pflanzen umwelt- und klimafreundlicher sein als die Gewinnung aus tierischen Quellen.

Proteine und im Besonderen Enzyme sind anfällig für physikalische und chemische Abbauprozesse und daher grundsätzlich instabil über längere Lagerungszeiten. Dies gilt auch für pflanzliche DAO, wobei sich die Instabilität des Enzyms DAO im Verlust der enzymatischen Aktivität zeigt, der über die Zeit größer wird.

Die Herstellung von pharmazeutischen Darreichungsformen und Nahrungsergänzungsmitteln, die pflanzliche DAO enthalten, erfordert folglich einen geeigneten Stabilisierungsschritt. Eine gängige Methode zur Stabilisierung von Enzymen und anderen biologischen Materialien ist die Lyophilisation oder Gefriertrocknung. Dieses Verfahren umfasst das Einfrieren der enzymhaltigen Lösungen, gefolgt von der Entfernung von Wasser durch Sublimation unter Vakuum bei niedrigen Temperaturen.

Allerdings kann auch die Lyophilisation zur Instabilität von Proteinen und Enzymen beitragen, da aufgrund der Phasenübergänge physikalischer Stress auf die Proteine ausgeübt wird. Hinzu kommt, dass Hilfsstoffe, die zur Stabilisierung der Proteinstruktur während der Lyophilisation dienen, und daher vor der Lyophilisation zugesetzt werden, nicht notwendigerweise auch die Stabilität der Proteine während der Langzeitlagerung (als Lyophilisat) gewährleisten, und teilweise diese sogar beeinträchtigen können.

Die häufigsten Mechanismen, die langfristig zu einem Proteinabbau führen sind Hydrolyse, Oxidation und Deaminierung. Als Gegenmaßnahmen werden häufig Stabilisatoren zum Schutz der Proteine zugesetzt. Die Wahl dieser Stabilisatoren hat einen großen Einfluss auf die Langzeitstabilität von Proteinen. Sie ist auch abhängig davon, welches Protein bzw. Enzym stabilisiert werden soll, da sich diese während der Lyophilisation bzw. der anschließenden Lagerung nicht notwendigerweise gleich verhalten.

Um die Langzeitstabilität der pflanzlichen DAO zu gewährleisten, müssen daher geeignete Stabilisatoren verwendet werden, die den bestmöglichen Schutz der pflanzlichen DAO sowohl während des Lyophilisationsprozesses, als auch während der Langzeitlagerung bieten.

Megoura et al. (Molecules 28 (2023): 992) beschäftigt sich mit der Verbesserung der Stabilität von DAO aus Erbsen (*Pisum sativum*) durch Trehalose bzw. Sucrose. Die DAO wurde in Abwesenheit oder in Anwesenheit von Saccharose oder Trehalose lyophilisiert und die Stabilität der erhaltenen Zubereitungen wurde während der Lagerung bei 4°C und -20°C über 18 Monate verfolgt. Aus den bei -20 °C gelagerten Lyophilisaten wurden außerdem durch direkte Kompression Tabletten formuliert. Die Tabletten wurden bei 4 °C gelagert und die Stabilität über 6 Monate verfolgt. Die mit Saccharose- oder Trehalose-Stabilisator gefriergetrockneten DAO-Pulver bzw. die daraus hergestellten Tabletten wiesen nach der Lagerung eine höhere Enzymaktivität auf, im Vergleich zu DAO-Pulver bzw. Tabletten, die keine Saccharose- oder Trehalose enthielten.

In dieser Studie wurden jedoch keine Stabilitätsuntersuchungen bei Raumtemperatur (25°C oder höher, beispielsweise 30°C) angestellt. Diese Lagerungsbedingungen sind jedoch von hoher praktischer Relevanz, da der logistische Aufwand, der mit der durchgehenden Kühlung von Arzneimitteln bzw. Nahrungsergänzungsmitteln einhergeht, mit hohen Kosten verbunden ist und daher in der Regel nicht akzeptabel ist. Hinzu kommt, dass aufgrund nicht-linearer Effekte auf die Proteinstabilität nicht in allen Fällen quantitative Rückschlüsse aus den bei deutlich niedrigeren Temperaturen gemachten Beobachtungen auf die Effekte von Stabilisatoren bei Raumtemperatur zulässig sind. Aufgrund des komplexen Wechselspiels zwischen den einzelnen möglichen Stabilisatoren und dem Enzym DAO ist eine Vorhersage darüber, welchen Effekt ein Hilfsstoff bzw. eine Klasse von Hilfsstoffen auf die Stabilität des Proteins haben wird, schwierig. Es besteht somit noch Bedarf nach einer weiteren Optimierung der Hilfsstoffe zur Stabilisierung von pflanzlicher DAO während und nach der Lyophilisierung, insbesondere bei anschließender Lagerung bei Raumtemperatur.

Somit besteht eine Aufgabe der vorliegenden Erfindung darin, ein Verfahren zur Herstellung eines stabilisierten Lyophilisats mit pflanzlicher DAO sowie ein entsprechendes stabilisiertes Lyophilisat zur Verfügung zu stellen, wobei das Lyophilisat auch bei praxisrelevanten Bedingungen (wie z.B. der Lagerung bei Raumtemperatur und/oder einer erhöhten Luftfeuchtigkeit) möglichst stabil bleiben soll, d.h. einen möglichst geringen Aktivitätsverlust aufweisen soll.

In einem ersten Aspekt, stellt die vorliegende Erfindung ein Verfahren zur Herstellung eines stabilisierten Lyophilisats mit pflanzlicher DAO zur Verfügung, das die folgenden Schritte umfasst:
- Bereitstellen von DAO-haltigen Pflanzen oder DAO-haltigen Teilen davon (insbesondere Pflanzensprossen);
- Homogenisieren der DAO-haltigen Pflanzen oder DAO-haltigen Teilen davon in wässriger Lösung, um ein wässriges Homogenisat zu erhalten;
- Hinzufügen von (a) einem Zucker oder Zuckeralkohol und (b) einer Aminosäure (diese dienen vorzugsweise als Stabilisatoren); und
- Lyophilisieren des wässrigen Homogenisats oder einer DAO-haltigen Fraktion davon in Anwesenheit von (a) dem Zucker oder Zuckeralkohol und (b) der Aminosäure.

Darüber hinaus stellt die vorliegende Erfindung ein stabilisiertes Lyophilisat mit pflanzlicher DAO zur Verfügung, das nach diesem Verfahren erhältlich ist.

In einem weiteren Aspekt stellt die vorliegende Erfindung ein stabilisiertes Lyophilisat mit pflanzlicher DAO zur Verfügung, umfassend (a) einen Zucker oder Zuckeralkohol und (b) eine freie Aminosäure. Diese beiden Komponenten dienen vorzugsweise als Stabilisatoren (mit anderen Worten liegen die Komponenten (a) und (b) vorzugsweise als Stabilisator vor).

Eine freie Aminosäure ist eine Aminosäure, welche nicht Teil eines Peptids oder eines Proteins ist.

Es wird des Weiteren eine feste pharmazeutische Darreichungsform (insbesondere eine Kapsel oder Tablette) zur Verfügung gestellt, welche eines der oben genannten erfindungsgemäßen Lyophilisate, gegebenenfalls in verpresster Form, beinhaltet. Diese Darreichungsform kann zur therapeutischen Verwendung, vorzugsweise in der Behandlung von Histamin-induzierten Störungen, herangezogen werden oder als Nahrungsergänzungsmittel oder diätetisches Lebensmittel verwendet werden.

Überraschenderweise wurde festgestellt, dass Kombinationen von einem Zucker oder Zuckeralkohol mit einer Aminosäure (insbesondere die in der Folge jeweils konkret offenbarten) besonders gut geeignet sind für die Stabilisierung der pflanzlichen DAO, insbesondere im Hinblick auf eine Langzeitlagerung, und insbesondere bei Raumtemperatur (und einer gewissen Luftfeuchtigkeit). Dabei hat sich herausgestellt, dass der Stabilisierungseffekt in Folge der Anwesenheit dieser beiden Komponenten additiv oder sogar synergistisch ist.

In einer bevorzugten Ausführungsform des erfindungsgemäßen Verfahrens ist die Aminosäure eine freie Aminosäure.

In einer bevorzugten Ausführungsform des erfindungsgemäßen Verfahrens oder des erfindungsgemäßen Lyophilisats wird die Aminosäure oder die freie Aminosäure bevorzugt ausgewählt aus der Gruppe, die aus basischen Aminosäuren wie Arginin, Histidin und Lysin sowie den Aminosäuren Asparagin, Glutamin, Cystein, Homocystein, Methionin, Tryptophan und Tyrosin besteht. Besonders bevorzugt sind basische Aminosäuren, insbesondere Arginin, Histidin und Lysin, bevorzugt Arginin.

Es versteht sich von selbst, dass die freie Aminosäure erfindungsgemäß auch in Form eines pharmazeutisch akzeptablen Salzes verwendet werden kann. Bei Arginin ist dies beispielsweise Argininhydrochlorid.

Eine weitere bevorzugte Ausführungsform des erfindungsgemäßen Verfahrens oder des erfindungsgemäßen stabilisierten Lyophilisats sieht vor, dass der Zucker oder Zuckeralkohol ausgewählt ist aus der Gruppe, die aus Disacchariden wie Trehalose, Sucrose, Lactose und Maltose, Trisacchariden wie Raffinose, Polysacchariden wie Maltodextrin und den Zuckeralkoholen Mannitol, Sorbitol, Glycerol, Xylitol, Erythritol und Inositol besteht. In einer besonders bevorzugten Ausführungsform ist der Zucker ein Disaccharid, insbesondere Trehalose.

In einer weiteren bevorzugten Ausführungsform des erfindungsgemäßen Verfahrens wird (a) der Zucker oder Zuckeralkohol bevorzugt in einer Menge von zumindest 10 Gew.-% des Homogenisats, mehr bevorzugt zumindest 20 Gew.-%, noch mehr bevorzugt zumindest 30 Gew.-%, insbesondere zumindest 40 Gew.-% oder gar zumindest 50 Gew.-% des Homogenisats und/oder (b) die Aminosäure oder die freie Aminosäure bevorzugt in einer Menge von zumindest 20 Gew.-% des Homogenisats, mehr bevorzugt zumindest 40 Gew.-%, noch mehr bevorzugt zumindest 60 Gew.-%, insbesondere zumindest 80 Gew.-% oder gar zumindest 100 Gew.-% des Homogenisats (auf Gewichtsbasis) als Stabilisator hinzugefügt.

In einer weiteren bevorzugten Ausführungsform des erfindungsgemäßen stabilisierten Lyophilisats mit pflanzlicher DAO umfasst das Lyophilisat (a) einen Zucker oder Zuckeralkohol in einem Gewichtsverhältnis von zumindest 1:1, bevorzugt von zumindest 4:1 oder gar zumindest 7:1, bevorzugt zumindest 10:1 oder gar zumindest 13:1, mehr bevorzugt zumindest 16:1 oder gar zumindest 19:1, insbesondere zumindest 22:1 oder gar zumindest 25:1 zum Gesamtproteingehalt des Lyophilisats, und/oder (b) eine freie Aminosäure in einem Gewichtsverhältnis von zumindest 1:1, bevorzugt von zumindest 4:1 oder gar zumindest 7:1, bevorzugt zumindest 10:1 oder gar zumindest 18:1, mehr bevorzugt zumindest 25:1 oder gar zumindest 32:1, insbesondere zumindest 40:1 oder gar zumindest 50:1 zum Gesamtproteingehalt des Lyophilisats.

Mit anderen Worten ist es bevorzugt, dass sowohl der Zucker oder Zuckeralkohol als auch die Aminosäure auf Gewichtsbasis im Überschuss zum Gesamtproteingehalt vorliegen.

In einer weiteren bevorzugten Ausführungsform des erfindungsgemäßen Verfahrens oder des erfindungsgemäßen stabilisierten Lyophilisats stammen die DAO-haltigen Pflanzen aus der Familie der Hülsenfrüchtler (Leguminosae), wobei sie bevorzugt aus den Gattungen *Pisum, Lens, Cicer, Lathyrus, Phaseolus* und *Vicia* ausgewählt werden. Diese Pflanzen (insbesondere deren Pflanzensprossen) stellen gute Quellen für eine pflanzliche DAO dar, die gemäß der vorliegenden Erfindung stabilisiert werden kann. Besonders bevorzugt sind hierbei Erbsen *(Pisum sativum),* Linsen (*Lens culinaris*)*,* Kichererbse (*Cicer arietinum*), Saat-Platterbsen (*Lathyrus* sativus), Bohnen (*Phaseolus vulgaris*) und Ackerbohnen (*Vicia faba*), insbesondere Erbsen (*Pisum sativum*)*.*

Pflanzensprossen sind das frühe Wachstumsstadium von Pflanzen, das kurz nach der Keimung der Samen beginnt. In diesem Stadium weisen Pflanzen eine hohe Konzentration an Nährstoffen, Vitaminen, Mineralstoffen und Enzymen (darunter DAO) auf, da sie für das schnelle Wachstum und die Entwicklung der jungen Pflanze benötigt werden. Daher sind die DAO-haltigen Pflanzen in einer bevorzugten Ausführungsform Sprossen.

In einer weiteren bevorzugten Ausführungsform des erfindungsgemäßen Verfahrens oder des erfindungsgemäßen stabilisierten Lyophilisats sind die DAO-haltigen Pflanzen Keimlinge.

In einer weiteren bevorzugten Ausführungsform des erfindungsgemäßen Verfahrens oder des erfindungsgemäßen stabilisierten Lyophilisats wird zumindest ein weiterer Stabilisator oder Hilfsstoff hinzugefügt. Die Zugabe dieser zusätzlichen Stabilisatoren kann die Stabilität des Lyophilisats weiter verbessern und gleichzeitig mögliche schädliche Reaktionen oder Oxidationsprozesse reduzieren, die die Qualität des Endprodukts beeinträchtigen könnten. Andere Hilfsstoffe wiederum können eine Vielzahl von Funktionen erfüllen, wie beispielsweise der Schutz des Proteins während des Einfrierens oder während des Trocknungsprozesses beim Lyophilisationsprozess oder die Verbesserung der Lyophilisierbarkeit oder die Verringerung von Aggregation im Lyophilisat (wenn es in vermahlener Form vorliegt). Als zusätzliche Stabilisatoren besonders bevorzugt sind Antioxidantien wie z.B. Lecithin, Tocopherol, Resveratrol, Milchsäure, Glutathion und/oder Ascorbinsäure und/oder Kryoprotektoren. Die zusätzlichen Stabilisatoren und/oder Hilfsstoffe können vor oder nach der Lyophilisation hinzugegeben werden. Wenn der Stabilisator ein Kryoprotektor ist, wird er vor der Lyophilisation zugegeben.

Gemäß einer weiteren bevorzugten Ausführungsform des erfindungsgemäßen Verfahrens umfasst der Herstellungsprozess des stabilisierten Lyophilisats zusätzlich das Vermahlen nach dem Lyophilisieren. Das Vermahlen kann insbesondere dann zum Einsatz kommen, wenn das Lyophilisat für die Weiterverarbeitung in Kapseln und Tabletten vorgesehen ist. Die feinere Partikelgröße erleichtert die Verarbeitung und ermöglicht eine gleichmäßige Verteilung des Wirkstoffs in der festen Darreichungsform. Dies führt zu einer konsistenteren Freisetzung des Wirkstoffs im Gastrointestinaltrakt und somit zu einer verbesserten Bioverfügbarkeit.

Nach dem Vermahlen kann das Lyophilisat in Kapseln gefüllt oder für die Herstellung von Tabletten, Pellets, Mikropellets, Granulat oder Jelly Beans verwendet werden. Bei der Herstellung dieser Darreichungsformen kann das vermahlene Lyophilisat beispielsweise mit weiteren Hilfsstoffen, wie Bindemitteln, Fließregulierungsmitteln oder Gleitmitteln, vermischt und anschließend gegebenenfalls verpresst werden, um die gewünschte Größe und Form zu erhalten.

Die erfindungsgemäße feste (pharmazeutische) Darreichungsform kann einen oder mehrere (pharmazeutisch akzeptable) Hilfsstoffe umfassen, beispielsweise einen Stoff, ausgewählt aus der Gruppe der Polysaccharide, wie Cellulose (z.B. mikrokristalline Cellulose), Hydroxypropylmethylcellulose, Carboxymethylcellulose, Hydroxyethylcellulose, Ethylhydroxyethylcellulose, Hydroxypropylcellulose, Celluloseacetylphthalat, Hydroxypropylmethyl-cellulosephthalat, Celluloseacetat, Cellulosepropionat, Celluloseacetatbutyrat, Ethylcellulose, Guar-Mehl, Alginsäure und/oder Alginate, Pektin, Polyvinylpyrrolidon, Polyvinylacetat, Polyvinylalkohol, Polyvinylchlorid, Polymere der Acrylsäure und deren Salze bzw. Polyacrylate bzw. Polymethacrylate bzw. Co-Polymere davon (z.B. Eudragit^{®} E, Eudragit^{®} R, Eudragit^{®} S, Eudragit^{®} NE, Eudragit^{®} RS, Eudragit^{®} RL), Vinylpyrrolidon-Vinylacetat-Copolymere, Schellack, Nylon, kolloidale Kieselsäure (z.B. Aerosil^{®}), Behenate (z.B. Glyceryl dibehenate), Stearate, Polyamid, Polyacrylamid, Polyethylen, Polyalkylenglykole wie Polyethylenglykol, Co-polymere von Polyalkylenglykolen, z.B. von Polyethylenglykol und Polypropylenglykol (Pluronic^{®}, BASF); und Gemischen davon. Insbesondere wenn die Darreichungsform eine Tablette ist, kann sie beispielsweise als Hilfsstoffe ein Füllmittel wie Lactose, ein Zerfallsmittel wie mikrokristalline Cellulose, ein Fließmittel wie kolloidale Kieselsäure und/oder ein Schmiermittel wie Magnesiumstearat enthalten.

Gemäß einer bevorzugten Ausführungsform ist die erfindungsgemäße Darreichungsform magensaftresistent, um eine gezielte Freisetzung der DAO im Darm zu ermöglichen. Magensaftresistenz kann beispielsweise mit Beschichtungen (z.B. der Tabletten, Kapsel oder Pellets) erreicht werden, die erst bei einem höheren pH-Wert, wie er im Darm vorliegt, aufgelöst werden. Dadurch wird erreicht, dass die in der Darreichungsform vorliegende, empfindliche DAO den Magen unbeschadet passiert. Beispiele für geeignete magensaftresistente Beschichtungen sind Polymere wie Polyvinylacetatphthalat (PVAP), Hydroxypropylmethylcellulosephthalat (HPMCP), Celluloseacetatphtalat (CAP), Celluloseacetatsuccinat, Hydroxypropylmethylcelluloseacetatesuccinat oder Schellack. Es können auch magensaftresistente Beschichtungen auf Basis von Methacrylsäurecopolymeren eingesetzt werden, wie z.B. Methylacrylatmethacrylsäure Copolymere oder Methylmethacrylatmethacrylsäure Copolymere. Ein weiteres bevorzugtes Polymer für eine magensaftresistente Beschichtung der erfindungsgemäßen Darreichungsform ist Glyceryl dibehenate. Die Darreichungsform kann z.B. in einer magensaftresistenten Form, wie beispielsweise in der WO 2006/003213 A1 offenbart, vorliegen.

In einer weiteren, bevorzugten Ausführungsform ist die erfindungsgemäße Darreichungsform eine Kapsel, eine Tablette oder ein Sachet. Wenn die Darreichungsform eine Kapsel ist, kann ihre Hülle beispielsweise im Wesentlichen aus Hartgelatine bestehen.

In einer weiteren Ausführungsform ist die erfindungsgemäße Darreichungsform eine retardierte Darreichungsform (d.h. eine Darreichungsform mit verzögerter Wirkstofffreisetzung) wie z.B. eine Retardtablette. Wie eine Darreichungsform in retardierter Form formuliert werden kann, ist im Stand der Technik bekannt, beispielsweise aus der WO 2010/028794 A1. Bevorzugt wird in der erfindungsgemäßen Ausführungsform ein Polymer als retardierender Hilfsstoff eingesetzt, welches Polymer ausgewählt ist aus den neutralen Homo- und Co-polymeren aus (Meth)acrylsäureestern, kationischen Homo- und Co-polymeren aus (Meth)acrylsäureestern mit quartären Ammoniumgruppen, Polyvinylacetat, Celluloseacetat, Cellulosepropionat, Celluloseacetatbutyrat und Ethylcellulose.

Wie bereits eingangs erwähnt kann über die Nahrung aufgenommenes, körperfremdes Histamin aber auch körpereigenes Histamin aufgrund von unerwünschten Reaktionen eine Vielfalt von Störungen auslösen, darunter Kopfschmerzen, Rhinitis, Tachykardie, Magen- und Darmbeschwerden und Hypotonie. Die Erfindung betrifft weiterhin die erfindungsgemäße feste Darreichungsform enthaltend das erfindungsgemäße Lyophilisat zur therapeutischen Verwendung, insbesondere zur Verwendung bei der Behandlung von Histamin-induzierten Krankheiten oder Störungen. Die DAO-Aktivität (angegeben in kilo Histamine Degrading Units (kHDU)/g Lyophilisat) kann zum Beispiel gemäß folgendem Messverfahren ermittelt werden: DAO REA ("DAO spezifischer radioextraction assay"). Besonders bevorzugt ist eine DAO-Aktivität im erfindungsgemäßen Lyophilisat von zumindest 5000 kHDU/g, bevorzugt zumindest 10000 kHDU/g, noch mehr bevorzugt zumindest 12500 kHDU/g, insbesondere zumindest 15000 kHDU/g oder gar zumindest 25000 kHDU/g. Üblicherweise liegt die Aktivität im erfindungsgemäßen Lyophilisat unter 100000 kHDU/g, inbesondere unter 80000 kHDU/g oder unter 70000 kHDU/g.

Der Gesamtproteingehalt im erfindungsgemäßen Lyophilisat kann durch Berechnung der Trocknungsausbeute und der Bestimmung des Proteingehalts im Erbsensprossenhomogenisat mit bekannten Methoden, wie zum Beispiel mittels BCA-Test (Bicinchoninsäure) (Smith, P.K. et al., Measurement of protein using bicinchoninic acid, Analytical Biochemistry 150(1): 76-85, 1985), der Dumas Methode (DIN EN ISO 16634-1:2009-07) oder der Kjeldahl Methode, bestimmt werden.

Die Erfindung betrifft ferner die folgenden Ausführungsformen:
Ausführungsform 1. Verfahren zur Herstellung eines stabilisierten Lyophilisats mit pflanzlicher Diaminooxidase (DAO), umfassend die folgenden Schritte:
   - Bereitstellen von DAO-haltigen Pflanzen oder DAO-haltigen Teilen davon;
   - Homogenisieren der DAO-haltigen Pflanzen oder DAO-haltigen Teilen davon in wässriger Lösung, um ein wässriges Homogenisat zu erhalten;
   - Hinzufügen von (a) einem Zucker oder Zuckeralkohol und (b) einer Aminosäure; und
   - Lyophilisieren des wässrigen Homogenisats oder einer DAO-haltigen Fraktion davon in Anwesenheit von (a) dem Zucker oder Zuckeralkohol und (b) der Aminosäure.
Ausführungsform 2. Verfahren gemäß Ausführungsform 1, wobei die Aminosäure eine freie Aminosäure ist.
Ausführungsform 3. Verfahren gemäß Ausführungsform 1 oder 2, wobei die Aminosäure ausgewählt ist aus der Gruppe bestehend aus basischen Aminosäuren wie Arginin, Histidin und Lysin, und den Aminosäuren Asparagin, Glutamin, Cystein, Homocystein, Methionin, Tryptophan und Tyrosin.
Ausführungsform 4. Verfahren gemäß Ausführungsform 3, wobei die Aminosäure eine basische Aminosäure ist.
Ausführungsform 5. Verfahren gemäß Ausführungsform 4, wobei die Aminosäure Arginin ist.
Ausführungsform 6. Verfahren gemäß einer der Ausführungsformen 1 bis 5, wobei der Zucker oder Zuckeralkohol ausgewählt ist aus der Gruppe bestehend aus Disacchariden wie Trehalose, Sucrose, Lactose, und Maltose, Trisacchariden wie Raffinose, Polysacchariden wie Maltodextrin, und den Zuckeralkoholen Mannitol, Sorbitol, Glycerol, Xylitol, Erythritol und Inositol.
Ausführungsform 7. Verfahren gemäß Ausführungsform 6, wobei der Zucker oder Zuckeralkohol ein Disaccharid ist.
Ausführungsform 8. Verfahren gemäß Ausführungsform 7, wobei der Zucker oder Zuckeralkohol Trehalose ist.
Ausführungsform 9. Verfahren gemäß einer der Ausführungsformen 1 bis 8, wobei die DAO-haltigen Pflanzen Mitglieder der Familie der Hülsenfrüchtler (Leguminosae) sind, bevorzugt ausgewählt aus den Gattungen *Pisum, Lens, Cicer, Lathyrus, Phaseolus* und *Vicia.*
Ausführungsform 10. Verfahren gemäß einer der Ausführungsformen 1 bis 9, wobei die DAO-haltige Pflanzen ausgewählt sind aus Erbsen (*Pisum sativum*)*,* Linsen (*Lens culinaris*)*,* Kichererbse (*Cicer arietinum*), Saat-Platterbsen (*Lathyrus* sativus), Bohnen (*Phaseolus vulgaris*) und Ackerbohnen (*Vicia faba*)*.* Ausführungsform 11. Verfahren gemäß Ausführungsform 10, wobei die DAO-haltigen Pflanzen Erbsen (*Pisum sativum*) sind. Ausführungsform 12. Verfahren gemäß einer der Ausführungsformen 1 bis 11, wobei die DAO-haltigen Pflanzen Sprossen oder Keimlinge sind.
Ausführungsform 13. Verfahren gemäß einer der Ausführungsformen 1 bis 12, wobei der Zucker oder Zuckeralkohol in einer Menge von zumindest 10 Gew.-% des Homogenisats, mehr bevorzugt zumindest 20 Gew.-%, noch mehr bevorzugt zumindest 30 Gew.-%, insbesondere zumindest 40 Gew.-% oder gar zumindest 50 Gew.-% als Stabilisator hinzugefügt wird.
Ausführungsform 14. Verfahren gemäß einer der Ausführungsformen 1 bis 13, wobei die Aminosäure in einer Menge von zumindest 20 Gew.-% des Homogenisats, mehr bevorzugt zumindest 40 Gew.-%, noch mehr bevorzugt zumindest 60 Gew.-%, insbesondere zumindest 80 Gew.-% oder gar zumindest 100 Gew.-% des Homogenisats als Stabilisator hinzugefügt wird.
Ausführungsform 15. Verfahren gemäß einer der Ausführungsformen 1 bis 14, wobei zumindest ein weiterer Stabilisator wie ein Antioxidans oder ein Kryoprotektor oder ein Hilfsstoff hinzugefügt wird, insbesondere Glutathion, Lecithin, Tocopherol, Resveratrol, Milchsäure und/oder Ascorbinsäure.
Ausführungsform 16. Verfahren gemäß einer der Ausführungsformen 1 bis 15, ferner umfassend das Vermahlen nach dem Lyophilisieren.
Ausführungsform 17. Verfahren gemäß einer der Ausführungsformen 1 bis 16, wobei (a) der Zucker oder Zuckeralkohol und/oder (b) die Aminosäure beim Hinzufügen als Stabilisator(en) hinzugefügt wird.
Ausführungsform 18. Stabilisiertes Lyophilisat mit pflanzlicher DAO, erhältlich nach dem Verfahren gemäß einer der Ausführungsformen 1 bis 17.
Ausführungsform 19. Stabilisiertes Lyophilisat mit pflanzlicher DAO, umfassend (a) einen Zucker oder Zuckeralkohol und (b) eine freie Aminosäure.
Ausführungsform 20. Stabilisiertes Lyophilisat nach Ausführungsform 18 oder 19, wobei der Zucker oder Zuckeralkohol (a) als Stabilisator dient.
Ausführungsform 21. Stabilisiertes Lyophilisat nach einer der Ausführungsformen 18 bis 20, wobei die freie Aminosäure (b) als Stabilisator dient.
Ausführungsform 22. Stabilisiertes Lyophilisat nach einer der Ausführungsformen 18 bis 21, wobei der Zucker oder Zuckeralkohol in einem Gewichtsverhältnis von zumindest 1:1, bevorzugt von zumindest 4:1 oder gar zumindest 7:1, bevorzugt zumindest 10:1 oder gar zumindest 13:1, mehr bevorzugt zumindest 16:1 oder gar zumindest 19:1, insbesondere zumindest 22:1 oder gar zumindest 25:1 zum Gesamtproteingehalt des Lyophilisats, vorliegt.
Ausführungsform 23. Stabilisiertes Lyophilisat nach einer der Ausführungsformen 18 bis 22, wobei die freie Aminosäure in einem Gewichtsverhältnis von zumindest 1:1, bevorzugt von zumindest 4:1 oder gar zumindest 7:1, bevorzugt zumindest 10:1 oder gar zumindest 18:1, mehr bevorzugt zumindest 25:1 oder gar zumindest 32:1, insbesondere zumindest 40:1 oder gar zumindest 50:1 zum Gesamtproteingehalt des Lyophilisats, vorliegt.
Ausführungsform 24. Stabilisiertes Lyophilisat nach einer der Ausführungsformen 18 bis 23, wobei die freie Aminosäure ausgewählt ist aus der Gruppe bestehend aus basischen Aminosäuren wie Arginin, Histidin und Lysin, und den Aminosäuren Asparagin, Glutamin, Cystein, Homocystein, Methionin, Tryptophan und Tyrosin.
Ausführungsform 25. Stabilisiertes Lyophilisat nach einer der Ausführungsformen 18 bis 24, wobei die freie Aminosäure eine basische Aminosäure ist.
Ausführungsform 26. Stabilisiertes Lyophilisat nach einer der Ausführungsformen 18 bis 25, wobei die freie Aminosäure Arginin ist.
Ausführungsform 27. Stabilisiertes Lyophilisat nach einer der Ausführungsformen 18 bis 26, wobei der Zucker oder Zuckeralkohol ausgewählt ist aus der Gruppe bestehend aus Disacchariden wie Trehalose, Sucrose, Lactose, und Maltose, Trisacchariden wie Raffinose, Polysacchariden wie Maltodextrin, und den Zuckeralkoholen Mannitol, Sorbitol, Glycerol, Xylitol, Erythritol und Inositol.
Ausführungsform 28. Stabilisiertes Lyophilisat nach einer der Ausführungsformen 18 bis 27, wobei der Zucker oder Zuckeralkohol ein Disaccharid ist.
Ausführungsform 29. Stabilisiertes Lyophilisat nach einer der Ausführungsformen 18 bis 28, wobei der Zucker oder Zuckeralkohol Trehalose ist.
Ausführungsform 30. Stabilisiertes Lyophilisat gemäß einer der Ausführungsformen 17 bis 29, wobei das Lyophilisat eine DAO-Aktivität von zumindest 1 kHDU/g, bevorzugt von zumindest 10 kHDU/g aufweist (vorzugsweise gemessen mit DAO-REA); vorzugsweise wobei die DAO-Aktivität im Lyophilisat bei zumindest 5000 kHDU/g, bevorzugt zumindest 10000 kHDU/g, noch mehr bevorzugt zumindest 12500 kHDU/g, insbesondere zumindest 15000 kHDU/g oder gar zumindest 25000 kHDU/g liegt (vorzugsweise gemessen mit DAO-REA).
Ausführungsform 31. Feste, vorzugsweise magensaftresistente Darreichungsform zur oralen Verabreichung, umfassend das stabilisierte Lyophilisat gemäß einer der Ausführungsformen 18 bis 30, gegebenenfalls in verpresster Form.
Ausführungsform 32. Feste Darreichungsform gemäß Ausführungsform 31 zur therapeutischen Verwendung, vorzugsweise in der Behandlung von Histamin-induzierten Störungen.
Ausführungsform 33. Verwendung der festen Darreichungsform gemäß Ausführungsform 31 als Nahrungsergänzungsmittel oder diätetisches Lebensmittel.

Im Folgenden wird die Erfindung anhand von bevorzugten, nicht einschränkenden Beispielen und Figuren näher erläutert.

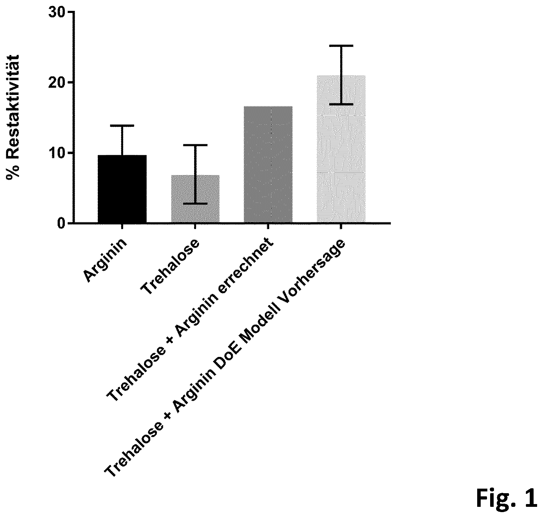

**Fig. 1****:** Restaktivität der DAO in einem lyophilisierten Erbsensprossen-Homogenisat gelagert bei 30°C, 65% relativer Luftfeuchtigkeit im offenen Behältnis versetzt mit den Zusätzen (jeweils im Gewichtsverhältnis Zusatz zu Homogenisat von 1:1): Arginin-Monohydrochlorid ("Arginin"), Trehalose ("Trehalose"), sowie die errechnete Restaktivität des lyophilisierten Erbsensprossen-Homogenisats bei Zusatz von Arginin-Monohydrochlorid und Trehalose in Kombination ("Trehalose + Arginin errechnet"). Im Vergleich dazu die zu erwartende, aus dem DoE-Modell vorhergesagte Restaktivität der Kombination von Arginin-Monohydrochlorid und Trehalose ("Trehalose + Arginin DoE Modell Vorhersage"). Die Fehlerindikatoren kennzeichnen das 95% Konfidenzintervall. Es zeigte sich ein Effekt von Arginin und Trehalose, der höher als erwartet war.

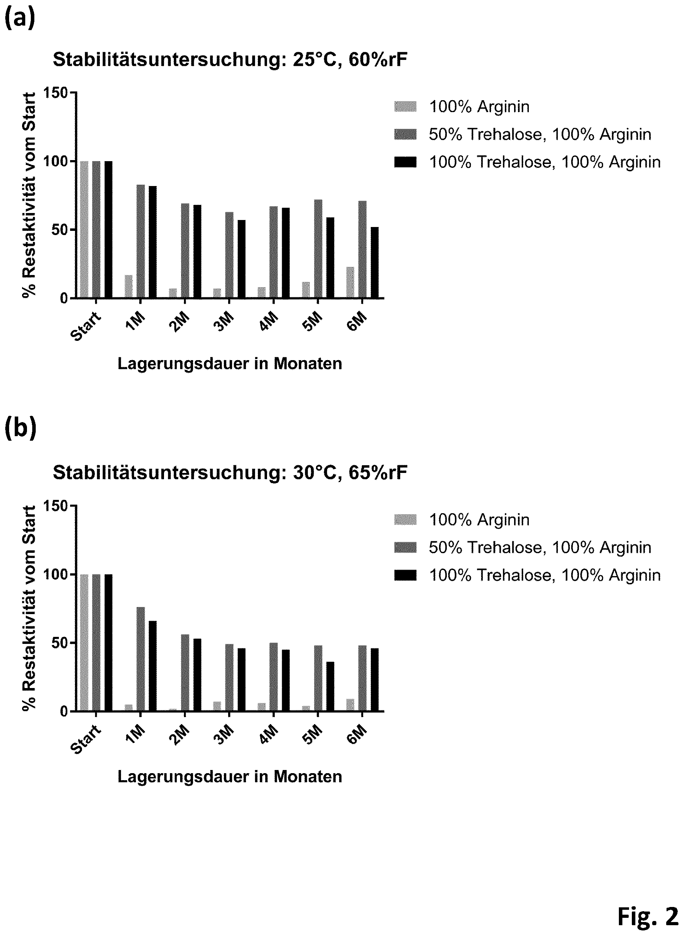

**Fig. 2****:** Stabilitätsuntersuchung der aus dem DoE-Modell abgeleiteten Erbsensprossen-Homogenisat-Formulierungen mit Arginin-Monohydrochlorid im Gewichtsverhältnis Arginin-Monohydrochlorid zu Homogenisat von 1:1 ("100% Arginin"), Trehalose (im Gewichtsverhältnis Trehalose zu Homogenisat von 1:2) und Arginin-Monohydrochlorid im Gewichtsverhältnis von 1:1 zum Homogenisat ("50% Trehalose, 100% Arginin") sowie Trehalose (im Gewichtsverhältnis Trehalose zu Homogenisat von 1:1) und Arginin-Monohydrochlorid im Gewichtsverhältnis von 1:1 zum Homogenisat ("100% Trehalose, 100% Arginin"). Die Daten zeigen den monatlichen Verlauf der DAO-Aktivitätsabnahme innerhalb einer Lagerungsdauer von 6 Monaten ("1M", "2M", "3M", "4M", "5M", "6M") mit Bezug auf den Startwert ("Start") als 100%. (a) Lagerung bei 25°C, 60% rF, (b) Lagerung bei 30°C, 65% rF.

### Beispiel 1 - Stabilitätsversuche

Es wurden Stabilitätsversuche mit pflanzlicher DAO und verschiedenen möglichen Stabilisatoren sowohl im Hinblick auf die Lyophilisierung als auch auf die anschließende Lagerung durchgeführt. Im Folgenden sind ausgewählte Versuche beschrieben.

Versuche mit Saccharose bzw. Maltodextrin als alleinige Stabilisatoren zeigten keine ausreichende Stabilisierung der lyophilisierten DAO aus Erbsensprossen (*Pisum sativum*) unter Langzeitlagerungsbedingungen bei 25°C und 60% relativer Luftfeuchtigkeit (rF).

In einem weiteren Versuch wurden zu einer DAO-reichen Erbsensprossen-Homogenisat-Fraktion mit 10% w/w Maltodextrin verschiedene Aminosäuren (bis zu 10% w/w) zugesetzt. Reduziertes Glutathion wurde ebenfalls zugesetzt, um als Antioxidans zu fungieren. Anschließend wurde das Homogenisat lyophilisiert. Nach der Lyophilisation waren im Lyophilisat 3-4 Gew% Erbsensprossen-Trockenmasse (hauptsächlich Erbsensprossenprotein, darunter auch die DAO) enthalten. Eine Langzeitlagerung (bis zu sechs Monate) bei 25°C, 60%rF zeigte, dass die Zugabe der Aminosäuren Cystein oder Methionin, in Kombination mit Maltodextrin zu einer verbesserten Langzeitstabilität der DAO im Pulver des Erbsensprossen-Homogenisats führte. Herausragende Ergebnisse (bis zu 60% Restaktivität der DAO nach 6 Monaten) wurden mit Arginin in Kombination mit dem Polysaccharid Maltodextrin erzielt. Auch mit Histidin in Kombination mit Maltodextrin wurden besonders gute Ergebnisse erzielt.

In einem weiteren Versuch wurde Trehalose als Stabilisator verwendet. Mittels eines Design of Experiments (DoE)-Ansatzes wurde gleichzeitig der Einsatz in Kombination mit Arginin und Maltodextrin geprüft, sowie unterschiedliche Konzentrationen der Mischungen untersucht. Um das Erbsensprossen-Homogenisat zu stabilisieren, wurde es mit Maltodextrin, Trehalose und Arginin in unterschiedlichen Gewichtsanteilen versetzt, lyophilisiert und bei 30°C, 65% rF in einem offenen Behältnis zur Stabilitätsbestimmung eingelagert. Formulierungen mit Arginin und Trehalose, aber ohne Maltodextrin, lieferten in diesem Versuchsansatz die besten DAO-Stabilitätswerte.

Dass es sich hierbei sogar um einen mehr als additiven Effekt handelte, war aus dem DoE-Modell ableitbar. Berechnet man die Summe der Restaktivität aus dem Einsatz von Trehalose nach 6-monatiger Lagerung bei 30°C, 65%rF im offenen Behältnis von 6,9% und der Restaktivität aus dem Einsatz von Arginin nach 6-monatiger Lagerung bei 30°C, 65%rF im offenen Behältnis von 9,7%, so ergibt sich ein errechneter Wert von 16,6% Restaktivität für eine Kombination aus Trehalose und Arginin. Der zu erwartende, aus dem DoE-Modell vorhergesagte Wert für die Kombination von Trehalose mit Arginin beträgt jedoch 21,1% (mit einem 95%igem Konfidenzintervall von 16,9% bis 25,2%). Folglich liegt ein synergistischer Effekt vor. Dies wird in Fig. 1 verdeutlicht.

Auf Basis der obigen Ergebnisse wurden drei Formulierungen ausgewählt, produziert und zur Verifizierung eingelagert. Die obigen Ergebnisse wurden dadurch bestätigt. In diesem Versuch ergab sich eine Restaktivität nach 6 Monaten von ca. 60% bei 25°C, 60%r.F, sowie von ca. 40% bei 30°C, 65%r.F., wenn als Formulierung 1:2 Trehalose zu Erbsensprossenhomogenisat und 1:1 Arginin (in Form des Monohydrochlorids von Arginin) zu Erbsensprossenhomogenisat bzw. 1:1 Trehalose zu Erbsensprossenhomogenisat und 1:1 Arginin (in Form des Monohydrochlorids von Arginin) zu Erbsensprossenhomogenisat gewählt werden (Angaben auf Gewichtsbasis). Wurde im Vergleich nur 1:1 Arginin (in Form des Monohydrochlorids von Arginin) zum Erbsensprossen Homogenisat zugesetzt, war die Aktivität der DAO bereits nach einem Monat auf unter 20% der Startaktivität gesunken. (siehe Fig. 2 a. und b.)

### Beispiel 2 - Herstellung der erfindungsgemäßen Dosierungsform

Es wurden DAO-haltige Erbsensprossen (*Pisum sativum*) bereitgestellt. Zur Herstellung des Erbsensprossen-Homogenisats wurden die gekeimten Erbsen mit einem ersten Teil Wasser in einem Mixer homogenisiert. Anschließend wurde das Homogenisat verdünnt, um die Pipettierbarkeit zu verbessern. Um Faserteile abzutrennen, wurde das Homogenisat bei 400xg für 15 Minuten zentrifugiert. Der Überstand wurde bei 11.000xg für 1 h 50min abermals abzentrifugiert und der Überstand sterilfiltriert. Dieser sterilfiltrierte Überstand war die Fraktion des Homogenisats, welche für die Lyophilisierung vorgesehen war. Die Bestimmung der DAO-Aktivität in den jeweiligen Verarbeitungsschritten zeigte, dass die Zentrifugations- und Filtrationsschritte keine Aktivitätseinbußen forderten.

Anschließend wird das so erhaltene Erbsensprossenhomogenisat mit einem Teil Arginin (100% w/w Arginin-Monohydrochlorid zu Erbsensprossenhomogenisat) und einem halben Teil Trehalose (50% w/w Trehalose zu Erbsensprossenhomogenisat) versetzt. Dies ergibt in Summe eine Mischung (auf Gewichtsbasis) aus 2/5 Erbsensprossenhomogenisat, 2/5 Arginin-Monohydrochlorid und 1/5 Trehalose. Diese Mischung wird eingefroren und anschließend lyophilisiert und vermahlen. Das Pulver wird mit folgenden Zusatzstoffen vermischt: Mikrokristaline Zellulose, Silizium Dioxid, Crosscarmellose Natrium, Magnesiumstrearat und tablettiert. Um eine Inaktivierung des Enzyms durch die Magenpassage zu verhindern, wird ein magensaftresistenter Überzug bestehend aus Schellack aufgebracht.

## Patentansprüche

1. Verfahren zur Herstellung eines stabilisierten Lyophilisats mit pflanzlicher Diaminooxidase (DAO), umfassend die folgenden Schritte:
- Bereitstellen von DAO-haltigen Pflanzen oder DAO-haltigen Teilen davon;
- Homogenisieren der DAO-haltigen Pflanzen oder DAO-haltigen Teilen davon in wässriger Lösung, um ein wässriges Homogenisat zu erhalten;
- Hinzufügen von (a) einem Zucker oder Zuckeralkohol und (b) einer Aminosäure; und
- Lyophilisieren des wässrigen Homogenisats oder einer DAO-haltigen Fraktion davon in Anwesenheit von (a) dem Zucker oder Zuckeralkohol und (b) der Aminosäure.

2. Verfahren gemäß Anspruch 1, wobei die Aminosäure ausgewählt ist aus der Gruppe bestehend aus basischen Aminosäuren wie Arginin, Histidin und Lysin, und den Aminosäuren Asparagin, Glutamin, Cystein, Homocystein, Methionin, Tryptophan und Tyrosin.

3. Verfahren gemäß Anspruch 2, wobei die Aminosäure eine basische Aminosäure, vorzugsweise Arginin, ist.

4. Verfahren gemäß einem der Ansprüche 1 bis 3, wobei der Zucker oder Zuckeralkohol ausgewählt ist aus der Gruppe bestehend aus Disacchariden wie Trehalose, Sucrose, Lactose, und Maltose, Trisacchariden wie Raffinose, Polysacchariden wie Maltodextrin, und den Zuckeralkoholen Mannitol, Sorbitol, Glycerol, Xylitol, Erythritol und Inositol.

5. Verfahren gemäß Anspruch 4, wobei der Zucker oder Zuckeralkohol ein Disaccharid, vorzugsweise Trehalose, ist.

6. Verfahren gemäß einem der Ansprüche 1 bis 5, wobei die DAO-haltigen Pflanzen Mitglieder der Familie der Hülsenfrüchtler (Leguminosae) sind, bevorzugt ausgewählt aus den Gattungen *Pisum, Lens, Cicer, Lathyrus, Phaseolus* und *Vicia.*

7. Stabilisiertes Lyophilisat mit pflanzlicher DAO, erhältlich nach dem Verfahren gemäß einem der Ansprüche 1 bis 6.

8. Stabilisiertes Lyophilisat mit pflanzlicher DAO, umfassend
(a) einen Zucker oder Zuckeralkohol und (b) eine freie Aminosäure.

9. Stabilisiertes Lyophilisat nach Anspruch 7 oder 8, wobei der Zucker oder Zuckeralkohol in einem Gewichtsverhältnis von zumindest 1:1, bevorzugt von zumindest 4:1 oder gar zumindest 7:1, bevorzugt zumindest 10:1 oder gar zumindest 13:1, mehr bevorzugt zumindest 16:1 oder gar zumindest 19:1, insbesondere zumindest 22:1 oder gar zumindest 25:1 zum Gesamtproteingehalt des Lyophilisats, vorliegt.

10. Stabilisiertes Lyophilisat nach einem der Ansprüche 7 bis 9, wobei die freie Aminosäure in einem Gewichtsverhältnis von zumindest 1:1, bevorzugt von zumindest 4:1 oder gar zumindest 7:1, bevorzugt zumindest 10:1 oder gar zumindest 18:1, mehr bevorzugt zumindest 25:1 oder gar zumindest 32:1, insbesondere zumindest 40:1 oder gar zumindest 50:1 zum Gesamtproteingehalt des Lyophilisats, vorliegt.

11. Stabilisiertes Lyophilisat nach einem der Ansprüche 7 bis 10, wobei die freie Aminosäure ausgewählt ist aus der Gruppe bestehend aus basischen Aminosäuren wie Arginin, Histidin und Lysin, und den Aminosäuren Asparagin, Glutamin, Cystein, Homocystein, Methionin, Tryptophan und Tyrosin; und/oder wobei der Zucker oder Zuckeralkohol ausgewählt ist aus der Gruppe bestehend aus Disacchariden wie Trehalose, Sucrose, Lactose, und Maltose, Trisacchariden wie Raffinose, Polysacchariden wie Maltodextrin, und den Zuckeralkoholen Mannitol, Sorbitol, Glycerol, Xylitol, Erythritol und Inositol.

12. Stabilisiertes Lyophilisat gemäß einem der Ansprüche 7 bis 11, wobei das Lyophilisat eine DAO-Aktivität von zumindest 1 kHDU/g, bevorzugt von zumindest 10 kHDU/g aufweist; vorzugsweise wobei die DAO-Aktivität im Lyophilisat bei zumindest 5000 kHDU/g, bevorzugt zumindest 10000 kHDU/g, noch mehr bevorzugt zumindest 12500 kHDU/g, insbesondere zumindest 15000 kHDU/g oder gar zumindest 25000 kHDU/g liegt.

13. Feste, vorzugsweise magensaftresistente Darreichungsform zur oralen Verabreichung, umfassend das stabilisierte Lyophilisat gemäß einem der Ansprüche 7 bis 12, gegebenenfalls in verpresster Form.

14. Feste Darreichungsform gemäß Anspruch 13 zur therapeutischen Verwendung, vorzugsweise in der Behandlung von Histamin-induzierten Störungen.

15. Verwendung der festen Darreichungsform gemäß Anspruch 13 als Nahrungsergänzungsmittel oder diätetisches Lebensmittel.
